Europäisches Patentamt

European Patent Office

Office européen des brevets

(19)

(11) Publication number: **0 205 071**

**A2**

(12)

# EUROPEAN PATENT APPLICATION

(21) Application number: **86107490.4**

(22) Date of filing: **03.06.86**

(51) Int. Cl.⁴: **C 12 N 15/00**

(30) Priority: **03.06.85 US 740604**

(43) Date of publication of application:
**17.12.86 Bulletin 86/51**

(84) Designated Contracting States:
**BE CH DE FR GB IT LI NL**

(71) Applicant: **THE JOHN HOPKINS UNIVERSITY**
**P.O. Box 2245R**
**Morristown, N.J. 07960(US)**

(72) Inventor: **Maier, Robert J.**
**1908 Fairbank Road**
**Baltimore Maryland 21209(US)**

(74) Representative: **Patentanwälte Grünecker, Kinkeldey,**
**Stockmair & Partner**
**Maximilianstrasse 58**
**D-8000 München 22(DE)**

(54) **Genes involved with hydrogenase and nitrogenase activities in rhizobium japonicum and cosmids.**

(57) Replicons are produced by combining a cloning vector and insert hup/nif DNA isolated from slow-growing species of *Rhizobium*, such as *Rhizobium japonicum*, and used to insert such hup/nif DNA into Hup⁻ and/or Nif⁻ species of *Rhizobium*.

**EP 0 205 071 A2**

# GENES INVOLVED WITH HYDROGENASE AND NITROGENASE ACTIVITIES IN RHIZOBIUM JAPONICUM AND COSMID VECTORS CONTAINING SAME

## Background of the Invention

### 1. Field of the Invention:

The present invention relates to nitrogen fixation by leguminous plants. More particularly, it relates to those genes involved in nitrogen fixation and the oxidation of hydrogen produced as a by-product of nitrogen fixation.

### 2. Description of the Prior Art:

The process of biological nitrogen fixation consists of the reduction of elementary nitrogen to ammonia by living organisms. This biologically fixed nitrogren is the major source of nitrogen available for the formation of protein, which is essential in maintaining the world food supply. The economic importance of biological nitrogen fixation on the Earth cannot be over emphasized. It has been reported that nitrogen fixation by all biological organisms accounts for 80 percent of the nitrogen fixed on Earth per year. Leguminous and non-leguminous symbionts alone account for about 5.5 million tons of nitrogen fixed per year in the United States. About 13,000 species of leguminous plants exist, the vast majority of which are involved in a symbiotic relationship with nitrogen fixing bacteria. Some of the economically more important legumes include soybean, peas, clover and the like. As a result of increased world population there has been increased activity in enhancement of world food sources. One food crop which in recent years has received ever increasing emphasis is soybean because of its high protein content. Much of the enhanced protein content of soybean results from the superior nitrogen fixing capabilities of Rhizobium japonicum the nitrogen fixing bacteria associated with soybean. Nitrogen fixation in R. japonicum is catalyzed by the enzyme nitrogenase. Nitrogenase catayses both the ATP-dependent reduction of $N_2$ to $NH_{4+}$

and protons to $H_2$. The evolution of $H_2$ by the nitrogenase reaction is a substantial energy loss during nitrogen fixation (Proc. Natl, Acad. Sci. USA 73:1207-1211, c1976). Some strains of R. japonicum express a membrane bound $H_2$-uptake hydrogenase system as bacteriods in soybean nodules (Arch fur Mikrobiol 85:193-201, c1972). In 1972, Dixon proposed that the $H_2$-uptake may be used to (i) reoxidize the $H_2$ evolved from nitrogenase for additional ATP and reductant for nitrogen fixation, (ii) utilize the excess $O_2$ to protect the oxygen labile nitrogenase, and (iii) prevent the $H_2$ inhibition of nitrogenase (Arch fur Mikrobiol 85:193-201, c1972). In both greenhouse and field studies, soybean plants inoculated with R. japonicum Hup+ strains have been shown to possess increased plant dry matter and increased amounts of nitrogen in seeds as compared to the plants inoculated with R. japonicum Hup⁻ strains (Agronomy Jour. 73:369-372, c1981 and 72:555-559, c1980).

The R. japonicum strains which possess an $H_2$ uptake capability in bacteriods can also utilize $H_2$ in free living culture when growing chemoautotrophically under microaerophilic conditions (Proc. Natl. Acad. Sci USA 76:1788-1792, c1979; J. Bacteriol. 141:664-670, c1980). Chemoautorophic growth conditions have been exploited to isolate numerous R. japonicum hydrogen-uptake (Hup) negative mutants (J. Bacteriol. 146:614-620, c1981 and 145:533-540, c1981). Biochemical and physiological characterization of some of these Hup mutants have helped to elucidate the components involved in $H_2$-uptake, and also how the system is regulated. Hydrogen oxidation activity is reconstitued when cell-free extracts from two different R. japonicum Hup⁻ mutants are combined (J. Biol. Chem. 257:2092-2096, c1982). Other mutants have $H_2$-uptake systems that are hypersensitive to repression by carbon substrates and $O_2$ (J. Bacteriol. 150:101-167, c1982). One of the most interesting classes of mutants are those which are defective in both nitrogenase (Nif⁻) and hydrogenase (Hup⁻) activities. Nif⁻ Hup⁻ mutant strain SR139 lacked activity for hydrogenase and both of the nitrogenase component proteins. However, reversion studies indicated that strain SR139 has only a single lesion which affected both nitrogenase and hydrogenase (J. Bacteriol. 155:926-929, c1983). Furthermore, this mutant strain produced nodules containing normal levels of leghemoglobin, and had a growth rate in free-living cultures like that of the parent strain. These results indicate the lesion is specific for the symbiotic properties of Nif and Hup. The isolation of Nif⁻ Hup⁻ mutants indicate that common biochemical and/or genetic components are involved in both nitrogen fixation and hydrogen oxidation.

Studies on the organization of genes responsible for $H_2$ oxidation in R. japonicum have been initiated. Cantrell et. al. (Proc. Natl. Acad. USA 80:181-185, c1983) have used an R. japonicum gene bank to isolate a $H_2$-uptake gene (hup) by complementation of an uncharacterized Hup⁻ mutant. When several hup gene cosmids were transferred by conjugation into a different Hup⁻ mutant, Hup+ transconjugants appeared at a low frequency. These results show that the cosmid may contain one hup gene and a part of another. Recently, Huagland et. al. (J. Bacteriol. 159:1006-1017, c1984) have reported that some hup genes of R. japonicum DES122 are linked in one area of its chromosomes, and organized in a minimum of two transcriptional units. They showed that approximately 15 kb of DNA of cosmid pHU1 are encoded for hup genes.

$H_2$ is oxidized by the hydrogenase enzyme and $CO_2$ is fixed by a RuBP carboxylase (Lepo, J. E., Hanus, F.J., and Evans, H. J., J. Bacteriol 141:664-670, c1980). Strains which possess such chemolithotrophic capacity may have an increased chance of survival in the soil where there is a limitation of fixed carbon substrates.

- 4 -

Highly active hydrogen-oxidizing systems have been found only in about 25% of R. japonicum strains and in no strain of R. trifolii or R. meliloti [Evans, H. J., Purohit, K., Cantrell, M. A., Eisbrenner, G., Russell, S. A., Hanus, F. J., & Lepo, J. E., Current Perspectives in Nitrogen Fixation, eds. Gibson, A. H. & Newton, W. E. (Elsevier/North Holland, New York, NY) 84-96]. Most strains of R. leguminosarum are Hup⁻ and with a few exceptions the Hup+ strains of this species recycle only a small proportion of the $H_2$ produced by the nitrogenase reaction (Nelson, L. M. and Salminen, S. O., J. Bacteriol. 151:989-995, c1982). It would be beneficial if Hup⁻ strains of any one of the several species of Rhizobium could be modified to Hup+, e.g. by a transfer of the genes for the Hup prototype from highly active Hup+ strains. Beneficial effects of the transfer of genes for the Hup phenotype could include not only the ability to utilize $H_2$ per se but also the ability to grow chemolithotrophically.

At least some of the determinants for the Hup phenotype have been shown to be plasmid-borne and transmissible in Alcaligenes eutrophus (Friedrich, B., Hogrefe, C., & Schlegel, H. G., J. Bacteriol. 147:198-205, c1981) and in R. leguminosarum (Brewen, N. J., DeJong, T. M., Phillips, D. A. and Johnston, A. W. B., Nature 288:77-79, c1980). In both R. leguminosarum and A. eutrophus, the Hup phenotype was carried on large, naturally occurring plasmids which must carry many other genes. The plasmid carrying Hup determinants in R. leguminosarum also was shown to carry determinants for nodulation specificity and for $N_2$ fixation. The host range for this plasmid may be limited (Brewen, N. J., DeJong, T. M., Phillips, D. A. & Johnston, A. W. B. Nature 28:77-79, c1980), and recipients of this plasmid may only be able to form effective nodules on pea plants.

Techniques for measurement of $H_2$ uptake have been described (Hanus, F. J., Carter, K. R. & Evans, H.

J., Methods Enzomol. 69:731-739, c1980).
(Bethlenfalvay, G. J. & Phillips, D. A., Plant Physiol.
63:816-820, c1979). Colonies of <u>Azotobacter</u> <u>chroococcum</u>
have been screened for expression of hydrogenase activ-
ity by observations of differences in rates of reduction
of methylene blue by Hup+ and Hup⁻ <u>Azotobacter</u> colonies
(Postgate, J. R., Partridge, C. D. P., Robson, R. L.,
Simpson, F. G., & Yates, M. G., J. Gen. Microbiol.
128:905-908, c1982). But none of these methods relates
to the isolation of Hup+ <u>Rhizobium</u> colonies from a large
number of putative recipients.

<u>SUMMARY OF THE INVENTION</u>:

This invention relates to transmissible DNA
regions containing DNA which code for all or a portion
of the genes of <u>R. japonicum</u> which code for the hydrogen
uptake and nitrogen fixation phenotypes. These replicons are referred herein as pSH DNA's. Specific
examples of such replicons described herein are pSH-1,
pSH-14 and pSH-22.

The following definitions may be useful in
understanding the terminology of this disclosure:

<u>Replicon</u> - A genetic element which is capable of independent replication. This may include a
plasmid, cosmid, or bacterophage DNA.

<u>Hup+</u> - A designation for the phenotype of <u>Rhizobium</u>
<u>japonicum</u> having the ability to enzymatically
uptake hydrogen·

<u>Hup-</u> - A designation for the phenotype of <u>Rhizobium</u>
<u>japonicum</u> which does not have the ability to
uptake hydrogen.

<u>hup</u> - A designation for a gene or a portion of a
which encodes for the uptake of hydrogen.

<u>hup DNA</u> - DNA which contains one or more hup genes or
portions of hup genes.

<u>Nif+</u> - A designation for the phenotype of <u>Rhizobium</u>
<u>japonicum</u> having the ability to fix
atmospheric nitrogen.

-6-

Nif-     - A designation for the phenotype of Rhizobium japonicum that does not have the ability to fix atmospheric nitrogen.

nif     - A designation for a gene or a portion of a gene which encodes for nitrogen fixation.

nif DNA     - DNA which containes one or more nif genes or a portion of such genes.

$SM^r$     - A designation for streptomycin resistance.

$Km^r$     - A designation for kanamyci resistance.

$Ry^r$     - A designation for refampin resistance.

Cfx     - A designation for carbon dioxide fixation activity (via ribulose biphosphate carboxylase).

Aut+     - Denotes the ability to grow chemoautotrophically.

$Tc^r$     - A designation for tetracyclin resistance.

nif/hup DNA     - A designation for DNA containing one or nif genes and one or more hup genes.

pSH-DNA     - A replicon produced by recombinant DNA techniques, and which contains hup DNA and nif DNA.

The object of this invention is to isolate and produce DNA which can be used to provide hydrogen uptake activity to Hup⁻ Rhizobium japonicum strains and to provide nitrogen fixing activity to Nif- Rhizobium japonicum strain. Such DNA may come from a strain of Rhizobium japonicum which contains hup genes and nif genes capable of showing recombination with these strains of R. japonicum thus converting them to the Hup+ on Nif+ phenotypes. The vector DNA to which the hup gene and nif gene containing DNA is attached may be a replicon which can be introduced and maintained in R. japonicum

## DESCRIPTION OF THE PREFERRED EMBODIMENTS

I. Materials

    A. Chemicals

Streptomycin sulfate, kanamycin sulfate, rifampicin, D-mannitol, Tween 40, sodium gluconate, alpha-ketoglutarate, and sodium EDTA were obtained from Sigma Chemical Co., St. Louis, MO. Tetracycline hydrochloride was purchased from Calbiochem-Behring Corp., La Jolla, CA. Yeast extract, Bacto-trytone, and agar were supplied by Difco Laboratories, Detroit, MI. $N_2$ and the gas mixture for autotrophic growth were obtained from Arundel Sales and Service Co., Baltimore, MD. Tris, SDS, phenol, agarose, chloroform and endonuclease restriction enzyme EcoRl was purchased from International Biotechnologies Inc., New Haven, CT. All other chemicals were reagent grade and were supplied by J. T. Baker Chemical, Phillipsburg, NJ.

B. Baoterial Strains, Plasmids or Gene Bank

R. japonicum strain SU306-47 was isolated as a Tn5-induced Hup⁻ mutant of strain USDA 110 Rif$^r$ (characterization of strain SU306-47 will be reported elsewhere). R. japonicum stock cultures were maintained on MYS agar plates supplemented with the appropriate antibiotic(s): streptomycin ($250\mu$g/ml); kanamycin (100 $\mu$ g/ml); rifampicin ($100\mu$g/ml); tetracycline (60 g/ml). E. coli stock cultures were maintained on LB agar plates supplemented with the appropriate antibiotic: tetracycline ($15\mu$g/ml); kanamycin ($50\mu$g/ml).

The R. japonicum DNA gene bank used in this study was supplied by G. Stacey, Univ. of Tennessee. R. japonicum I-110 DNA was partially digested with restriction endonuclease EcoRl and ligated into cosmid pLAFRl. The average insert size was approximately 20 kb.

The bacterial strains and plasmids used in this study are listed in the following Table I.

### TABLE I

| Strain or Plasmid | Relevent Genotype or Phenotype | Reference |
|---|---|---|
| R. japonicum | | |
| a. SR | $Sm^r$ $Km^r$ Hup+ | 1 |
| b. SR139 | $Sm^r$ $Km^r$ Hup⁻ Nif⁻ | 2 |
| c. SR114 | $Sm^r$ $Km^r$ Hup⁻ | 3 |
| d. SR118 | $Sm^r$ $Km^r$ Hup⁻ | 3 |
| e. SR119 | $Sm^r$ $Km^r$ Hup⁻ | 4 |
| f. SR122 | $Sm^r$ $Km^r$ Hup+ Cfx⁻ | 3 |
| g. SR129 | $Sm^r$ $Km^r$ Hup⁻ | 4 |
| h. SR140 | $Sm^r$ $Km^r$ Hup⁻ | 3 |
| i. SR143 | $Sm^r$ $Km^r$ Hup⁻ Nif⁻ | 2 |
| j. SR146 | $Sm^r$ $Km^r$ Hup⁻ | 5 |
| k. SR152 | $Sm^r$ $Km^r$ Hup⁻ | 3 |
| l. SR166 | $Sm^r$ $Km^r$ Hup⁻ | 3 |
| m. SR174 | $Sm^r$ $Km^r$ Hup⁻ | 4 |
| n. SR178 | $Sm^r$ $Km^r$ Hup⁻ | 4 |
| o. SR180 | $Sm^r$ $Km^r$ Hup⁻ | 4 |
| p. SR186 | $Sm^r$ $Km^r$ Hup⁻ | 4 |
| q. SR192 | $Sm^r$ $Km^r$ Hup⁻ | 3 |
| r. SU | $Rif^r$ Hup+ (derivative of USDA 110) | 6 |
| s. SU306-47 | $Rif^r$ (Tn5 induced) Hup⁻ | This Study |
| E. coli | | |
| a. HB101 | pro leu thi lacy $Sm^r$ endo I⁻ | 7 |
| Plasmids | | |
| a. pLAFR1 | $Tc^r$ | 8 |
| b. pKR2013 | $Km^r$ | 9 |

- 9 -

## References

1. Maier, R. J., J. R. Postgate, and H. J. Evans. 1978. _Rhizobium japonicum_ mutants unable to use hydrogen. Nature (London) 276:494-496.

2. Moshiri, F., L. Stults, P. Novak, and R. J. Maier. 1983. Nif⁻ Hup⁻ Mutants of _Rhizobium japonicum_. J. Bacteriol. 155:926-929.

3. Maier, R. J. 1981. _Rhizobium japonicum_ mutant strains unable to grow chemoautotrophically with $H_2$. J. Bacteriol. 145:533-540.

4. Maier, R. J. and D. M. Merberg. 1982. _Rhizobium japonicum_ mutants that are hypersensitive to repression of $H_2$ uptake by oxygen. J. Bacteriol. 150:161-167.

5. Maier, R. J. and S. Mutaftschiev. 1982. Reconstitution of $H_2$ oxidation activity from $H_2$ uptake-negative mutants of _Rhizobium japonicum_ bacteriods. J. Biol. Chem. 257:2092-2096.

6. Hom, S. S. M., S. L. Uratsu, and F. Hoang. 1984. Transposon Tn5-induced mutagenesis of _Rhizobium japonicum_ yielding a wide variety of mutants. J. Bacteriol. 159:335-340.

7. Boyer, H. W. and D. Roulland-Dussoix. 1969. A complementation analysis of the restriction and modification of DNA in _Escherichia coli_. J. Mol. Biol. 41:459-472.

8. Freidman, A. M., S. R. Long, S. E. Brown, W. J. Buikema, and F. M. Ausubel. 1982. Construction of a broad host range cosmid cloning vector and its use in the genetic analysis of _Rhizobium_ mutants. Gene 18:289-296.

9. Ditta, G., S. Stanfield, D. Corbin, and D. R. Helinski. 1980. Broad host range DNA cloning system for Gram negative bacteria: construction of a gene bank of _Rhizobium meliloti_. Proc. Natl. Acad. Sci., USA 77:7347-7351.

## II. Methods

### A. Media and Growth Conditions

Rhizobium japonicum was routinely grown in either the mannitol/yeast extract (MYS) medium described by Lim and Shanmugam (Biochem. Biophys. Acta. 584:479-492, c1979) or the yeast extract medium (YE) described by Hom et. al. LB medium was used as described by Miller to cultivate E. coli (J. Bacteriol. 145:533-540, c1981). Since Stults and co-workers have recently shown that nickel is an integral component of hydrogenase in R. japonicum SR (J. Bacteriol. 159:153-158, c1984), BM-no carbon medium was supplemented with $5 \mu$m $NiCl_2$. After inoculation, agar plates (1.5% Noble agar) were placed in gas-tight 20 liter capacity polyethylene buckets. The container was first sparged with $N_2$ and then with a gas mixture consisting of 10% $H_2$, 5% $CO_2$, 1% $O_2$, And 84% $N_2$. The buckets were incubated at 30°C for 7-14 days and resparged with the gas mixture every 2 days.

### B. Bacterial Filter Mating Technique

Late log phase E. coli donor cells and mid-log phase R. japonicum recipient cells ($10^9$ each) were mixed together, collected onto a metrical membrane filter (25 mm diameter, $0.45 \mu$m pore size; Gelman Instrument Co., Ann Arbor, Mich.), and incubated on YE agar plates at 30°C for 5 days. The cells were then resuspended in 5 ml of Tween 40 [0.01% (vol/vol)], serially diluted, and aliquots were spread onto the appropriate selection plates. The plates were incubated at 30°C for 7-14 days. The titer of recipients at the time of plating onto the selective medium was determined by spreading aliquots onto mannitol/glutamate agar plates, and incubated at 30°C for 7 days.

### C. Determination of the Minimum Inhibitory Concentration (MIC)

The MIC is defined as the lowest antibiotic concentration at which no colonies appeared. Mid-log phase R. japonicum cells were cultivated and aliquots containing 100-300 cells were spread onto MYS agar
plates containing increased levels of tetracycline as
described (J. Bacteriol. 159:335-340, c1984).

    D.   Hydrogen-Uptake Induction and Assay

       $H_2$-uptake activity of free-living cultures of
R. japonicum was measured by the induction procedure of
Maier (J. Bacteriol. 145:533-540, c1981) with minor
modifications.  After growth in YE (supplemented with 50
$\mu$g/ml tetracycline when tranconjugants were cultured)
to an $OD_{540}$ of 0.5-0.7, the bacteria were harvested by
centrifugation (1500 x g;10 min) at room temperature and
resuspended in 0.05 M potassium phosphate buffer (pH 7.0)
containing 2.5 mM $MgCl_2$.  Then 10 ml of cells ($OD_{540}$ of
0.5) were dispensed into 160 ml serum bottles (No.
223748; Wheaton, Millerville, NJ).  Serum bottles were
sealed with rubber stoppers (13 x 20 mm) fitted with
aluminum collars, and then sparged with 10% $H_2$, 5% $CO_2$,
And 84% $N_2$.  The bottles were incubated at 30°C for 48
hours on a rotary shaker (100 rpm; Model G-24, New
Brunswick Scientific, Edison, NJ).

       $H_2$-uptake specific activities were measured
amperometrically as described (20, 30) except that the
amperometric chamber size was 4.5 ml.  Cell numbers were
estimated by utilizing a standard curve of colony
forming units versus optical density at 540 nm.

    E.  Soybean Nodulation and Nitrogenase Assays

       Rhizobium japonicum strains were checked for
effectiveness (nitrogen fixation) with Essex soybean
(Glycine Max, L. Merr).  Seeds were surface sterilized
by immersion first with 95% ethanol and then with 0.2%
$HgCl_2$, rinsed 10 times with sterile distilled $H_2O$, and
incubated in the dark for 4 days at 30°C on the surface
of 1% water agar plates, as described by Vincent
(Vincent, J. M. "A manual for the practical study of
root-nodule, bacteria.  International Biological Program,
Blackwell Scientific Publications.  Oxford, England.
1970).  Rhizodium japonicum cultures for seed inoculation were grow in MSY medium as described previously (J. Bacteriol. 159:335-340, c1984). This medium for growing transconjugants was supplemented with 50 $\mu$g/ml tetracycline. Germinated soybean seeds were inoculated with $10^9$ Rhizobium japonicum cells. Soybean plants were grown for 6 weeks by the pouch method as described (Science 201:448-450, c1978). Uninoculated control plants did not form nodules.

Nitrogenase activities were measured by placing root sections containing nodules into a 160 ml serum bottle. After sealing the bottles with rubber stoppers fitted with aluminum collars, acetylene was added to a final concentration of 10% (vol/vol). After incubating for 30 minutes at room temperature, acetylene reduction (ethylene formation) was determined by gas chromatography as described (J. Bacteriol. 159:335-340, c1984). Acetylene reduction activity was linear through this time period.

Rhizobium japonicum strains responsible for infection were reisolated from nodules by the nodule surface sterilization procedure of Vincent, which utilized 95% ethanol and 3% hydrogen peroxide. Nodules were crushed onto MSY agar plates containing 60 $\mu$g/ml tetracycline. These agar plates were incubated at 30°C for 10 days.

F.   Nitrogenase Induction and Assay

Nitrogenase activity in free-living cultures of R. japonicum was induced by the procedure of Agarwal and Keister (Appl. Environ. Microbiol. 45:1592-1601, c1983). Tetracycline (50 $\mu$g/ml) was included in the induction culture of R. japonicum transconjugants. Prescription bottles (250 ml vol) were used instead of 500 ml reagent bottles. Acetylene (10% vol/vol) was added at the beginning of the incubation period. Nitrogenase activities and estimation of cell numbers were measured as described in the previous section.

G.   Cosmid DNA Isolation and Bacterial Transformations

Cosmid DNA from SR139 transconjugants was iso-

lated as described by Kado and Lui (13), with modifica-
tions. Rhizobium japonicum transconjugants were grown
in YE + 50µg.ml tetracycline to late log phase, washed
twice with 40 mM Tris-acetate containing 2 mM EDTA pH
7.9 (E buffer) plus 3% NaCl (wt/vol), and once with E
buffer minus 3% NaCl to remove the exopolysacharides.
The final cell pellet was resuspended in 0.5 ml E
buffer, and the cells were lysed with 1.0 ml of 3% SDS
(wt/vol) in E buffer pH 12.6. After incubation at 65°C
for 1 h, the preparation was extracted with 1.5 ml of
unbuffered phenol-chloroform (1:1) and centrifuged at
8000 xg for 15 min. The DNA in the aqueous phase was
precipitated by adding 1/10 vol of 2.5 M sodium acetate
pH 5.2 and 2 vol of cold ethanol. After incubation at
-20°C overnight, the DNA was harvested by centrifugation
for 15 min in a microfuge at 4°C. After drying the DNA
pellet, the DNA was resuspended in 6 mM Tris, 0.1 mM
EDTA, 10 mM NaCl pH 7.4.

Competent E. coli HB101 cells were prepared by
the procedure of Maniatis [Maniatis, T., E. F. Fritsch,
and J. Sambrook. Molecular cloning (a laboratory
manual). Cold Spring Harbor Laboratory, Cold Spring
Harbor, NY. 1982.]

III. Results

A. Complementation of an R. japonicum Nif- Hup-
Mutant with an R. japonicum Gene Bank

The Nif⁻ Hup⁻ mutant, strain SR139, was con-
jugated with the pLAFR1 cosmid clone library. Rhizobium
japonicum colonies able to grow autotrophically (Aut+)
with hydrogen and carbon dioxide as sole energy and carbon source were selected. In filter matings between E.
coli donors containing the R. japonicum I-110 clone bank
and SR139 recipients, the frequency of Aut+ colonies per
recipient (3.2 x 10⁻⁶) was two times greater than that
of spontaneous SR139 Aut+ revertants (1.6 x 10⁻⁶). The
Aut+ strains were isolated and then screened for growth
on MSY agar plates containing tetracycline (60 µg/ml).
Out of 156 Aut+ colonies tested, twenty-seven tetracycline-resistant (TC$^r$) strains were isolated. Three randomly chosen Aut+ Tc$^r$ strains [SR139 (pSH7), SR139 (pSH14), SR139 (pSH20)] possessed substantially increased tetracycline resistance compared to SR139. The single cell minimum inhibitory concentration (MIC) for tetracycline for these three strains was > 150 $\mu$ g/ml, which was at least three times greater than that of the SR139 parent strain (45 $\mu$ g/ml). Moreover, all Tc$^r$ Aut+ isolates possessed the stretomycin and kanamycin resistances of the SR139 parent. These results suggest that the Tc$^r$ Aut+ strains are SR139 transconjugants that harbor a clone containing gene which complemented the Hup$^-$ phenotype.

    B.    <u>Hydrogen-Uptake and Nitrogenase Activities in Putative SR139 Transconjugants</u>

Reversion analysis of strain SR139 had previously shown that the genetic defect which resulted in the Hup$^-$ Nif$^-$ phenotype was a single mutation (J. Bacteriol. 155:926-929, c1983). SR139 revertants able to grow autotrophically were shown to possess both hydrogen-uptake and symbiotic nitrogenase activities. To further established positive complementation of both defects of SR139, oxygen-dependent H$_2$-uptake activities were measured amperometrically on whole cells of SR139 Tc$^r$ Aut+ transconjugates. The results are set forth in the following Table II.

## TABLE II

### Hydrogen Oxidation Activities of SR139 Transconjugants

| Strain | Hydrogen-uptake Activity[a] (nmol $H_2$ oxidized/h/$10^8$ cells) |
|---|---|
| SR | 136 |
| SR139 | 0 |
| SR139 | 0 |
| SR139 | 201 |
| SR139 | 156 |
| SR139 | 102 |
| SR139 | 45 |
| SR139 | 78 |
| SR139 | 53 |
| SR139 | 84 |
| SR139 | 158 |
| SR139 | 76 |
| SR139 | 82 |
| SR139 | 83 |
| SR139 | 62 |
| SR139 | 112 |
| SR139 | 93 |
| SR139 | 153 |
| SR139 | 45 |
| SR139 | 68 |
| SR139 | 126 |
| SR139 | 76 |
| SR139 | 84 |
| SR139 | 110 |
| SR139 | 79 |
| SR139 | 91 |
| SR139 | 69 |
| SR139 | 131 |

[a] Cells were grown, harvested, resuspended in 0.05 M potassium phosphate buffer, and depressed for 48 h in an atmosphere containing 84% $N_2$, 10% $H_2$, 5% $CO_2$, and 1% $O_2$ as described in the methods section. Oxygen-dependent hydrogen-uptake specific activities were determined amperometrically as described in the methods sections.

As is apparent from Table II, all transconjugants possessed high levels of $H_2$-uptake specific
activities. That the various transconjugants contain
$H_2$-uptake activities that range from 30 to 150% of wild
type should not be seen as unusual because variability
in $H_2$ oxidation rates even in a single strain after
depression has been previously observed.

Secondly, nitrogenase activities (acetylene
reduction assays) were measured on root sections containing nodules from soybean plants that had been inoculated with six transconjugates. These six
transconjugants were chosen for analyses because casmido
that were isolated from item complemented strain SR139
at a requency of 1.0. The results are as set forth in
the following Table III.

## TABLE III

Symbiotic Nitrogenase Activities of SR139 Transconjugants

| Strain | Hydrogen-uptake Activity[a] (umole $C_2H_2$ reduced/h/gmn fresh nodule wt) |
|---|---|
| SR | 17.8 |
| SR139 | 0 |
| SR139 (pLAFR1) | 0 |
| SR139 (pSH15) | 0.59 |
| SR139 (pSH16) | 1.02 |
| SR139 (pSH17) | 0.52 |
| SR139 (pSH19) | 1.06 |
| SR139 (pSH22) | 0.50 |
| SR139 (pSH26) | 0.22 |

[a] Six week old soybean plants were harvested and nodu-
les on root sections were assayed for acetylene
reduction as described in the methods section.

As is apparent from Table III modules from plants inoculated with the transconjugates possessed nitrogenase specific activities approximately 1-6% of that for the wild type SR strain.  On the other hand, nodules from plants inoculated with SR139 or SR139 (pLAFR1) did not have nitrogenase activity.

Tetracycline-resistant Hup+ strains have been reisolated from nodules from soybean plants that had been inoculated with these R. japonicum transconjugates.

C.    Analysis of Cosmids Containing the Nif/Hup Gene

Cosmid DNA from fifteen SR139 transconjugants was isolated and transferred to E. coli HB101 by transformation.  There cosmids were generally designated pSH. A representative example of these cosmids pSH-22 is obtainable from Escherichia coli HB101, NRRL number B-15906.  This biologically pure culture is available from the permanent collection of the Northern Regional Research Laboratories, Agricultural Research Services, US Department of Agricultural under the above accession number, Peoria, Illinois, USA.  The permanancy of the deposit of the culture, and ready accessbility thereto by public are afforded throughout the effective life of the patent in the event the patent is granted.  Access to the culture is available during the pendency of the application under 37 CFR1.14 and 35 USC§112.  All restrictions on the availability to the public of the deposited culture will be irrevocably removed upon granting a patent.  The nif/hup cosmids are unique because they contain a nif/hup gene that is involved in both nitrogenase and hydrogenase activities.  The specific vector DNA used (pLaFR1) is not itself new.  Other vector DNA may be similarly useable so long as it can be easily introduced or maintained in R. japonicum and a bacterium such as E. coli commonly used in genetics laboratories.  The hup DNA and nif DNA contained in the cosmid is the insert DNA which produces the Hup+ and Nif+ character.  The hup/nif DNA may come from any organism which contains hup and nif genes, but preferably is derived from R. japonicum. The characteristics of the
nur-nif DNA listed below should not be construed as
limiting, nor need the functional characteristic be specifically associated, with DNA fragments having the
molecular sizes listed in Table IV. Cosmid DNA from
the HB101 transformants was digested with restriction
endonuclease EcoRl and DNA restriction fragments were
separated by agarose gel electrophoresis. Using conventional techniques, the restriction pattern resulting
from the gel electrophoresis for each of the transconjugants resulted in a number of common DNA fragments,
the approximate molecular size of which in kilobase
pairs are set forth in the following Table IV.

## TABLE IV

| Fragment | Molecular size (kb) |
|---|---|
| 1 | 21.6 |
| 2 | 13.2 |
| 3 | 4.0 |
| 5 | 3.0 |
| 6 | 2.5 |

The 21.6 kilobase fragment which co-migrated with
the linearized PLAFR1 vector was derived from the vector, and therefore is not a fragment of hup DNA or
nif-DNA.

The DNA used as the source of the hup/nif DNA was
orignally digested with the restriction enzymes EcoRI.
Accordingly the hup and nif DNA can be easily cleaved
from the vector, PLAFR1, by partial digestions with
EcoRI using procedures well known in the art. The isolated hup and nif DNA can be joined with different vectors and used to insert such hup and nif DNA into other
Hup⁻ and/or Nif⁻ bacteria species also using conventional vectors and methods to.

To show the presence of the nif/hup gene in
cosmid clones, randomly chosen representatives cosmids,
having the restriction pattern of Table IV, were transferred via conjugation from E. coli HB101 SR139 (Hup⁻
Nif⁻) using conventional conjugation techniques as for example those described in *Maniatis [Maniatis, et.al., ibid]* , and evaluated for their ability to grow under autotrophic conditions using the procedures described in *J. Bacteriol, 145: 533-540. 1981* . The results of these evaluations are set forth in the following Table V.

## TABLE V

### Complementation of SR139 with Recombinant Cosmids containing the Nif/Hup Gene[a]

| E. coli donor[b] | $Tc^r$/Recipient | Aut+/$Tc^r$ |
|---|---|---|
| HB101 (pSH15) | $1.0 \times 10^{-3}$ | 1.0 |
| HB101 (pSH16) | $8.1 \times 10^{-4}$ | 1.0 |
| HB101 (pSH17) | $7.2 \times 10^{-4}$ | 1.0 |
| HB101 (pSH19) | $1.0 \times 10^{-3}$ | 1.0 |
| HB101 (pSH22) | $1.8 \times 10^{-4}$ | 1.0 |
| HB101 (pSH26) | $9.8 \times 10^{-4}$ | 1.0 |
| HB101 (pSH25) | $5.8 \times 10^{-4}$ | $<2.0 \times 10^{-2}$ |
| HB101 (pLAFR1) | $1.0 \times 10^{-4}$ | $<1.0 \times 10^{-2}$ |

[a] Recipient for all matings was the Nif⁻ Hup⁻ strain SR139.

[b] HB101 (pRK2013) was included in all mating to mobi-

As is apparent from the results set forth in Table V, all $Tc^r$ SR139 had the ability to grow under autotrophic conditions and were according Hup+ phenotype.

Randomly selected $Tc^r$ Hup+ SR139 transconjugants from each mating were evaluated to determine whether they possessed whole cell (ex planta) nitrogenase activity. The procedure employed is described in *Appl. Envirom. Microbiol, 345: 1592-1601. 1983* . The results of these studies are set forth in the following Table VI.

## TABLE VI

### Ex planta Nitrogenase Activity of SR139 Transconjugants
### Obtained from 1:1 Complementation Studies

| Strain | Nitrogenase Activity[a] (nmol $C_2H_2$ reduced/h/$10^9$ cells) |
|---|---|
| SR | 3.70 |
| SR139 | 0 |
| SR139 (pLAFR1) | 0 |
| SR139 (pSH15) | 1.19 |
| SR139 (pSH16) | 1.21 |
| SR139 (pSH17) | 1.18 |
| SR139 (pSH19) | 1.69 |
| SR139 (pSH22) | 1.71 |
| SR139 (pSH26) | 1.36 |

[a]  Culture were induced and assayed for nitrogenase
    activity as described in the methods sections.

As shown in Table VI, randomly selected Tc[r] Hup+ SR139
transconjugants from each mating possessed whole cell
(ex planta) nitrogenase activities.  Transconjugant
nitrogenase activities were 30-50% of the value obtained
for the parent strain SR.  SR139 And SR139 (pLAFR1) did
not express nitrogenase activity.

    E.    Transfer of a Cosmid containing the Nif/Hup
        Gene into Other R. japonicum Hup⁻ Mutants

Since the nif/hup gene cosmid possessed an
insert of approximately 23 kb, there existed the possibility that other genes involved in the expression of
hydrogenase were encoded on this recombinant cosmid.  A
series of experiments were conducted to determine the
nature of the genes on the insert.  In these experiments,
representative cosmid pSH22, which complemented Nif⁻
Hup⁻ SR139 was transferred by conjugation from E. coli
HB101 to fifteen other Hup⁻ mutants using the procedures
of Maniatis, ibid.

. In previous studies, the nature of the defect causing a Hup⁻ phenotype was shown to differ amongst the various Hup⁻ mutants. Tetracycline-resistant transconjugants were isolated using the procedure ~~of~~ described in the methods section, , and then screened for their $H_2$-uptake capability by their growth response under autotrophic conditions using the procedure of Maier J. Bacteriol. 145: 533-540 1981. . The results are set forth in Table VII.

## TABLE VII

### Complementation of R. japonicum Hup- Mutants with a Cosmid Containing the Nif/Hup Genea

| Recipient | Phenotype | $TC^r$/Recipient | $Aut^+/Tc^r$ |
|---|---|---|---|
| SR114 | Hup- | $2.4 \times 10^{-5}$ | 1.0 |
| SR118 | Hup- | $6.0 \times 10^{-5}$ | 1.0 |
| SR119 | Hup- | $1.0 \times 10^{-4}$ | 1.0 |
| SR140 | Hup- | $2.2 \times 10^{-5}$ | 1.0 |
| SR146 | Hup- | $4.2 \times 10^{-5}$ | 1.0 |
| SU 306-47 | Hup- | $8.5 \times 10^{-6}$ | 1.0 |
| SR122[b] | Cfx- | $3.1 \times 10^{-5}$ | $<1.0 \times 10^{-5}$ |
| SR129 | Hup- | $1.9 \times 10^{-4}$ | $<1.0 \times 10^{-2}$ |
| SR143 | Nif-  Hup- | ND[c] | $<1.0 \times 10^{-2}$ |
| SR152 | Hup- | $1.8 \times 10^{-5}$ | $<1.0 \times 10^{-2}$ |
| SR166 | Hup- | $3.0 \times 10^{-4}$ | $<1.0 \times 10^{-2}$ |
| SR174 | Hup- | $2.6 \times 10^{-3}$ | $<1.0 \times 10^{-2}$ |
| SR178 | Hup- | $2.9 \times 10^{-4}$ | $<1.0 \times 10^{-2}$ |
| SR180 | Hup- | $1.3 \times 10^{-4}$ | $<1.0 \times 10^{-2}$ |
| SR186 | Hup- | $7.5 \times 10^{-3}$ | $<1.0 \times 10^{-2}$ |
| SR192 | Hup- | $4.8 \times 10^{-5}$ | $<1.0 \times 10^{-2}$ |

a   The recombinant cosmid used in all matings was pSH22 harbored in HB101.

b   SR122 is a derivative of SR that lacks RuBP carboxylase activity (18).

c   Not determined.

As is apparent from the results set forth in Table VII SR114, SR118, SR119, SR140, SR146, and SU 306-47 appeared at a frequency of 1.0 per cosmid transfer. All of these mutants are Hup⁻ but Nif⁺. The results show that the nif/hup cosmid pSH22 as well as other cosmids having the restriction pattern on Table IV contains at least one and perhaps other hup genes which are involved in $H_2$ uptake activity exclusively. When nif/hup cosmid pSH22 was transferred to 15 R. japonicum Hup⁻ Nif⁺ mutants. Hup⁺ transconjugants were detected at a frequency of

1.0 per cosmid transfer for mutant strains SR114, SR118, SR119, SR140, SR146, and SU306-47. These six Hup⁻ Nif⁺ mutants do not possess any whole-cell $H_2$ uptake activity when oxygen or methylene blue is used as the electron acceptor (Maier, R. J, J. Bacteroil 145; 533-540 (1981). Moreover, the hydrogenase polypeptides which were detected in bacteroid extracts of the wild type after two-dimensional protein polyacrylamide gel analysis were not detected in any of these six mutnats (E. B. O'Hara, Ph.D. thesis, Johns Hopkins University, Baltimore, MD., 1984).

On the other hand, some of the R. japonicum mutants that were not complemented by nif/hup cosmid pSH22 possessed different characteristics. For example, unlike strain SR139, strain SR143 was a Nif⁻ Hup⁻ mutant that was deficient in the production of heme (Moshers, F. L., et.a., J. Bacteriol 155; 926-929 (1983). Therefore, the lesion in this mutant is not directly related to nif or hup. Another Hup⁻ mutant that was not complemented by the nif/hup cosmid pSH22 was strain SR166. Strain SR166 lacked whole-cell $H_2$ uptake activity when it was coupled to oxygen, and this Hup⁻ mutant possessed Hup activity when methylene blue or phenazine methosulfate was used as an electron acceptor (Maier, R. J. Ibid). Strain SR166 apparently retains a functional hydrogenase, but it may lack an electron transport component(s) operating betwen hydrogenase and the terminal electron acceptor, oxygen (Maier, R. J. Ibid). Another mutant not complemented by the nif/hup cosmid was strain SR122. Strain SR122 is Hup⁺, but it is Aut⁻ due to a deficiency in ribulose bisphosphate carboxylase activity (Maier, R. J. ibid). Finally, some other, uncharacterized Hup⁻ strains were not complemented. These results suggest that other distinct genetic determinants for the simultaneous expression of nitrogenase and hydrogenase activities, electron transport components for $H_2$ oxidation, carbon dioxide fixation, and $H_2$ uptake are not on nif/hup cosmid pSH22. These genetic determinants may be

0205071

located on the DNA sequences adjacent to the ends of the nif/hup DNA insertion or may be unlinked.

The successful completion of two types of R. japonicum mutants (i.e., Nif⁻ Hup⁻ and Hup⁻) suggests that nif/hup recombinant cosmids such as pSH22 may contain a cluster of genes involved in $H_2$ uptake activity and at least one gene also involved with both of the nitrogenase components proteins.

WHAT IS CLAIMED IS:

1. Cosmid pSH 22 which is present in the culture of _Escherichia coli_ HB101 having deposit accession number NRRL B-15906,

2. A replicon which can be maintained in a slow-growing Rhizobium species that is useful for nitrogen fixation and hydrogen uptake in leguminous plants, said replicon comprising a cloning vector joined with hup/nif DNA necessary for hydrogenase and nitrogenase activity; said hup/nif DNA of the type present in cosmid pSH22.

3. A replicon according to claim 2 wherein said species is _Rhizobium japonicum_.

4. A replicon according to claim 3 wherein the hup/nif DNA comprises one or more of the fragments identified in Table IV.

5. A cosmid containing the nif/hup DNA included in pSH22 present in the culture of _E. coli_ HB101 having deposit accession number NRRL B-15906, or containing nif/hup DNA which is equivalent to that contained in pSH22.

6. A replicon according to claim 2 wherein said cloning vector is PLARF1.

7. A biologically pure culture of _E. Coli_ HB101 containing the cosmid pSH-22 or a cosmid containing nif/hup DNA which is the equivalent to the nif/hup DNA present in cosmid pSH-22.

8. A process for converting $Nif^-$ and/or $Hup^-$ _Rhizobium_ phenotypes which comprises inserting a cosmid pSH22, or a cosmid containing nif/hup DNA which is equivalent to the nif/hup DNA present in pSH22 into cells of said phenotype.

9. The process of claim 8 wherein said _Rhizobium_ is a slow growing _Rhizobium_.

10. The process of claim 9 wherein said _Rhizobium_ is _R. japonicum_.